(19) **Europäisches Patentamt**

**European Patent Office**

**Office européen des brevets**

(11) **EP 0 816 867 B1**

(12) **EUROPEAN PATENT SPECIFICATION**

(45) Date of publication and mention
of the grant of the patent:
**01.12.1999  Bulletin 1999/48**

(51) Int Cl.6: **G01S 15/89**

(21) Application number: **97304320.1**

(22) Date of filing: **19.06.1997**

(54) **Transducer element alignment structure in two dimensional array transducer for forming ultrasonic three dimensional images**

Anordnungsstruktur der Wandler-Elemente in einem zweidimensionalen Transducer für dreidimensionale Bilder

Structure de positionnement de transducteurs élémentaires dans un transducteur bidimensionel pour créer des images tridimensionelles

(84) Designated Contracting States:
**DE FR GB IT NL**

(30) Priority: **25.06.1996  KR 9623662**

(43) Date of publication of application:
**07.01.1998  Bulletin 1998/02**

(73) Proprietor: **MEDISON CO., LTD.**
**Kangwon-do 250-870 (KR)**

(72) Inventor: **Song, Tai-Kyong**
**Mapo-gu Seoul 121-742 (KR)**

(74) Representative: **Read, Matthew Charles et al**
**Venner Shipley & Co.**
**20 Little Britain**
**London EC1A 7DH (GB)**

(56) References cited:
EP-A- 0 214 782      DE-A- 4 405 504
US-A- 4 530 363      US-A- 4 586 512
US-A- 5 329 496

- SMITH S W ET AL: "HIGH-SPEED ULTRASOUND VOLUMETRIC IMAGING SYSTEM-PART I: TRANSDUCER DESIGN AND BEAM STEERING" IEEE TRANSACTIONS ON ULTRASONICS, FERROELECTRICS AND FREQUENCY CONTROL, vol. 38, no. 2, 1 March 1991, pages 100-108, XP000178890
- SHUNG K K ET AL: "ULTRASONIC TRANSDUCERS AND ARRAYS" IEEE ENGINEERING IN MEDICINE AND BIOLOGY MAGAZINE, vol. 15, no. 6, November 1996, pages 20-30, XP000638025

## Description

The present invention relates to a transducer array for use in three dimensional ultrasonic scanning.

Recently, ultrasonic diagnostic systems capable of providing three-dimensional images have been developed. Such systems might use mechanical means to scan in three-dimensions. However, these systems are complicated and require long scanning times. In fact, scanning times are so long that the image is influenced by the respiration of a patient. A solution might be for the patient to stop breathing while an ultrasonic image is taken, but the scanning times are too long. It is also difficult to obtain a reliable images due to the beating of the heart.

To reduce the three-dimensional scanning time, a two-dimensional array transducer can be used. However, such a system becomes very complicated because several thousands of transducer elements are need to make up the two-dimensional array.

It has been proposed to make the transducer with a circular array of transducer elements, arranged in concentric rings, with the elements on each ring separated by the same length of arc. The transducer element pattern will exhibit n-fold rotational symmetry, where n is the largest number of elements common to all rings. Such a device is shown in US-4586512. The present invention seeks to improve upon this configuration of elements.

According to the present invention there is provided a transducer array comprising first and second concentric rings of transducer elements for use in three dimensional ultrasonic scanning wherein a radial line passing through a first of the elements on the first ring does not pass through any of the elements on the second ring.

The transducer array may include a third concentric ring of transducer elements, wherein the radial line does not pass through any of the elements on the third ring. The third ring may also be disposed radially outwardly of the first and second rings. The concentric rings may be separated by equal radial distances and for at least one of the rings, the elements thereof may be separated by the same circumferential length. Accordingly, the circumferential length may be the same for all rings.

An ultrasonic imaging apparatus may include transducer elements.

A preferred embodiment of the present invention will now be described by way of example, with reference to the following drawings wherein:

Figure 1 shows a transducer element alignment structure for use in a two-dimensional array transducer according to the present invention;

Figure 2 shows a two-dimensional transducer in which 499 elements are aligned using the alignment structure shown in Figure 1; and

Figures 3A and 3B show graphs of field response with respect to different values of RES and CES.

The polar field response of an array of transducers will show a main lobe, a side lobe and a grating lobe. The elements should be aligned so that the field of each element is randomly added near the main lobe rather than increasing the side lobe due to a random array of transducer elements. In the present invention, the elements are randomly aligned on circular grids thereby reducing a grating lobe while simultaneously preventing the level of the side lobe from rising.

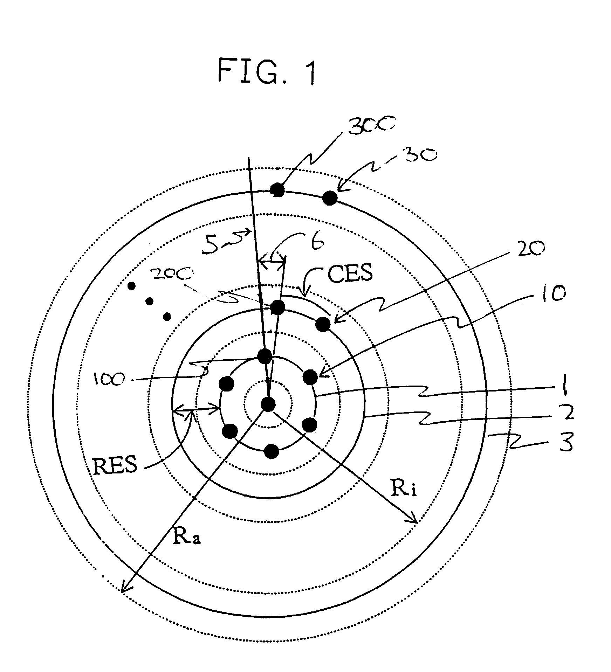

Figure 1 shows an element alignment structure for use in a two-dimensional array transducer.

Referring to Figure 1, first, second and third, and outermost, rings 1, 2, 3 are arranged concentrically. On the first ring 1, a first set of transducer elements 10 are aligned, starting from a first transducer element 100. On the second ring 2, a second set of transducer elements 20 are aligned, starting from a second transducer element 200. Similarly, on the third, and outermost, ring 3, a third set of transducer elements 30 are aligned, starting from a third transducer element 300. The spacing between a ring and its neighbour is the same for all rings and is marked RES. The spacing between each element on a ring is the same for all rings and is marked CES. CES is circumferential length between adjacent elements. A radial line 5, passes through the centre of the first transducer element 100. The second and third transducer elements 200, 300 are placed on their respective rings such that neither of them lie on the radial line 5. Thus, the second transducer element 200 is offset by a length of arc 6.

Circular apertures are indicated by dotted lines. One circular grid is separated from another according to a predetermined relationship. A circular aperture having a radius Ra is composed of Nc circles or dotted lines having the following equation:

$$R_i - R_{i-1} = 2.R_i = \text{constant} \ (i = 3, 4, ..., Nc)$$

where, $R_i$ is the radius of the i-th circular aperture.

The elements are located on the circles indicated by a solid line between the circular apertures. A first element is aligned to a location on each circle and the other elements are aligned to locations such that the circumferential distance between the elements are of the same length of arc. The distance between the elements on each circle is called the circular element spacing (CES). The distance between circles is called the radial element spacing (RES) and is a uniform distance.

In the element alignment structure of a two-dimensional array transducer shown in Figure 1, the first element is aligned to any location on each grid to obtain a smaller grating lobe. Since the elements uniformly distributed in the radial direction, the size of the side lobe does not increase.

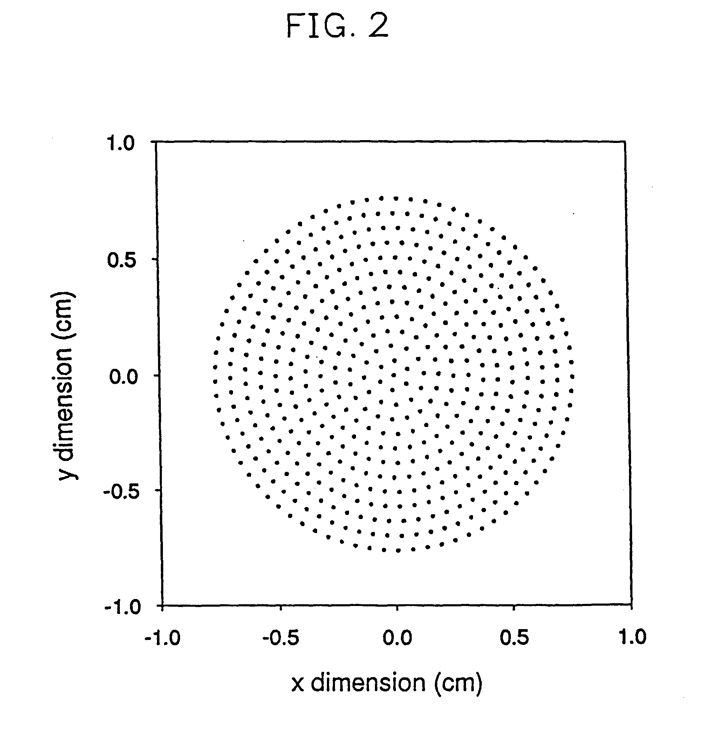

Figure 2 shows a two-dimensional transducer in which 499 elements are aligned using the alignment structure shown in Figure 1.

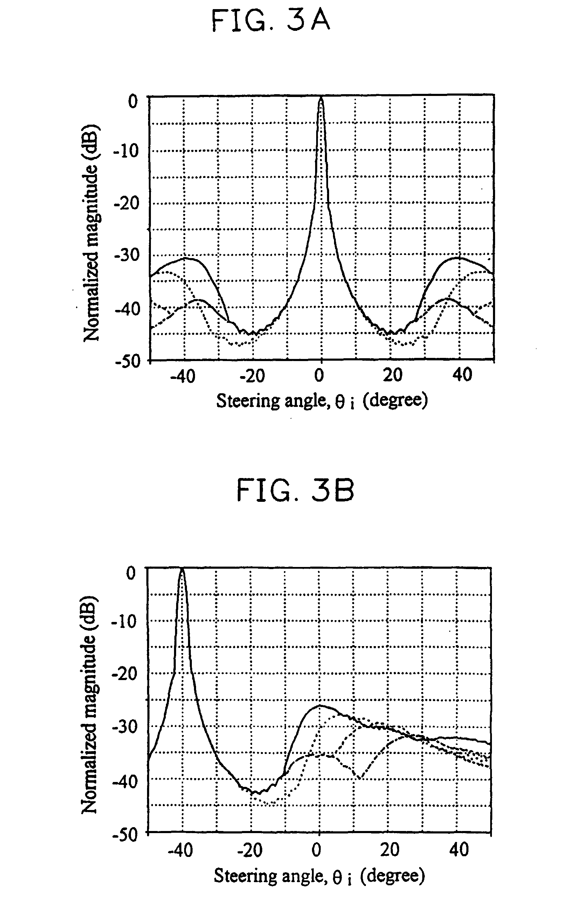

Figures 3A and 3B show graphs of field response for a two-dimensional array transducer using the element alignment structure with different values of RES and CES. Figures 3A and 3B show the field response when a main lobe is located at an angle of 0° and -40° respectively.

The solid lines in Figures 3A and 3B show field responses of the beams with respect to the array transducer composed of 499 elements having a RES of 1.4λ and a CES of 1.2λ when the centre frequency is 3.5 MHz and the diameter is 1.584 cm which corresponds to 38λ. The dotted lines show field responses of the beams with respect to the array transducer composed of 673 elements having a RES of 1.4λ and a CES of 1.2λ. The intermittent solid lines show field responses of the beams with respect to the array transducer composed of 703 elements having a RES of 1.2λ and a CES of 1.0λ. The dotted and intermittent lines show field responses of the beams with respect to the transducer composed of 877 elements having a RES of 1.4λ and a CES of 0.8λ.

Figures 3A and 3B show that as the RES is reduced, the size of the side lobe is also decreased. The size of the grating lobe is decreased according to the CES value. Thus, the values of RES and CES are controlled through the element alignment structure, to reduce the magnitude of side lobe and the grating lobe. Also, the array transducer comprised of 499 elements has a smaller grating lobe than that of the array transducer comprised of 625 elements.

As described above, the element alignment structure reduces the number of the transducer elements needed in a two-dimensional array transducer to effectively form ultrasonic three-dimensional images.

It will be appreciated that many modifications can be made to the embodiments described above.

For a discussion of how an array of ultrasonic transducer elements can be used to develop a three dimensional image, reference is directed to our co-pending WO 94/28467. This discloses an array of ultrasonic transducer elements and processing circuitry for developing a focused image from the array.

**Claims**

1. A transducer array comprising first and second concentric rings (1, 2) of transducer elements (10, 20) for use in three dimensional ultrasonic scanning characterised in that a radial line (5) passing through a first of the elements (100) on the first ring does not pass through any of the elements on the second ring.

2. A transducer array according to claim 1 including a third concentric ring (3) of transducer elements (30), wherein the radial line (5) does not pass through any of the elements on the third ring.

3. A transducer array according to claim 2 wherein the third ring (3) is disposed radially outwardly of the first and second rings (1, 2).

4. A transducer array according to claim 2 or 3 wherein the concentric rings (1, 2, 3) are separated by equal radial distances (RES).

5. A transducer array according to any preceding claim wherein for at least one of the rings (1, 2, 3) the elements (10, 20, 30) thereof are separated by the same circumferential length (CES).

6. A transducer array according to claim 5 wherein the circumferential length (CES) is the same for all rings (1, 2, 3).

7. An ultrasonic imaging apparatus including an array of transducer elements in accordance with any preceding claim.

**Patentansprüche**

1. Meßwandler-Anordnung, mit einem ersten und einem zweiten konzentrischen Ring (1, 2) aus Meßwandlerelementen (10, 20) zur Verwendung in einer dreidimensionalen Ultraschallabtastung, dadurch gekennzeichnet, daß eine durch ein erstes der Elemente (100) auf dem ersten Ring verlaufende radiale Linie (5) nicht durch irgendeines der Elemente auf dem zweiten Ring verläuft.

2. Meßwandler-Anordnung nach Anspruch 1, mit einem dritten konzentrischen Ring (3) aus Meßwandler-Elementen (30), wobei die radiale Linie (5) nicht durch irgendeines der Elemente auf dem dritten Ring verläuft.

3. Meßwandler-Anordnung nach Anspruch 2, wobei der dritte Ring (3) radial außerhalb des ersten und des zweiten Rings (1, 2) angeordnet ist.

4. Meßwandler-Anordnung nach Anspruch 2 oder 3, wobei die konzentrischen Ringe (1, 2, 3) durch gleiche radiale Strecken (RES) getrennt sind.

5. Meßwandler-Anordnung nach irgendeinem vorangehenden Anspruch, wobei für wenigstens einen der Ringe (1, 2, 3) dessen Elemente (10, 20, 30) durch dieselbe Umfangslänge (CES) getrennt sind.

6. Meßwandler-Anordnung nach Anspruch 5, wobei die Umfangslänge (CES) für sämtliche Ringe (1, 2, 3) gleich ist.

7. Ultraschall-Abbildungsvorrichtung, die eine Anordnung aus Meßwandlerelementen nach irgendeinem vorangehenden Anspruch enthält.

**Revendications**

1. Groupement de transducteurs comprenant des premier et deuxième anneaux concentriques (1, 2) d'éléments transducteurs (10, 20) pour utilisation en balayage ultrasonore tridimensionnel, caractérisé en ce qu'une ligne radiale (5) passant par un premier des éléments (100) du premier anneau ne passe par aucun des éléments du second anneau.

2. Groupement de transducteurs selon la revendication 1, incluant un troisième anneau concentrique (3) d'éléments transducteurs (30), dans lequel la ligne radiale (5) ne passe par aucun des éléments du troisième anneau.

3. Groupement de transducteurs selon la revendication 2, dans lequel le troisième anneau (3) est disposé radialement vers l'extérieur des premier et deuxième anneaux (1, 2).

4. Groupement de transducteurs selon la revendication 2 ou 3, dans lequel les anneaux concentriques (1, 2, 3) sont séparés par des distances radiales égales (RES).

5. Groupement de transducteurs selon l'une quelconque des revendications précédentes, dans lequel, pour au moins l'un des anneaux (1, 2, 3), ses éléments (10, 20, 30) sont séparés par la même distance circonférentielle (CES).

6. Groupement de transducteurs selon la revendication 5, dans lequel la distance circonférentielle (CES) est la même pour tous les anneaux (1, 2, 3).

7. Appareil de formation d'image à ultrasons incluant un groupement d'éléments transducteurs selon l'une quelconque des revendications précédentes.

# FIG. 1

# FIG. 2

## FIG. 3A

## FIG. 3B